(19) Europäisches Patentamt
European Patent Office
Office européen des brevets

(11) Veröffentlichungsnummer: **0 436 232 A1**

(12) **EUROPÄISCHE PATENTANMELDUNG**

(21) Anmeldenummer: 90125806.1

(51) Int. Cl.⁵: **A61F 9/00**

(22) Anmeldetag: **29.12.90**

(30) Priorität: **05.01.90 DE 4000181
21.09.90 DE 4030004**

(43) Veröffentlichungstag der Anmeldung:
**10.07.91 Patentblatt 91/28**

(84) Benannte Vertragsstaaten:
**AT BE CH DE DK ES FR GB GR IT LI LU NL SE**

(71) Anmelder: **Hermeking, Heino, Dr.**
**Voltaweg 15**
**W-4000 Düsseldorf 13(DE)**

(72) Erfinder: **Hermeking, Heino Dr**
**Voltaweg 15**
**W-4000 Düsseldorf 13(DE)**
Erfinder: **Dieter Komma**
**Am Leimbach**
**W-6909 Dielheim 3(DE)**

(74) Vertreter: **Meyer-Roedern, Giso, Dr.**
**Bergheimer Strasse 10-12**
**W-6900 Heidelberg 1(DE)**

(54) **Instrument zum Implantieren einer künstlichen Linse.**

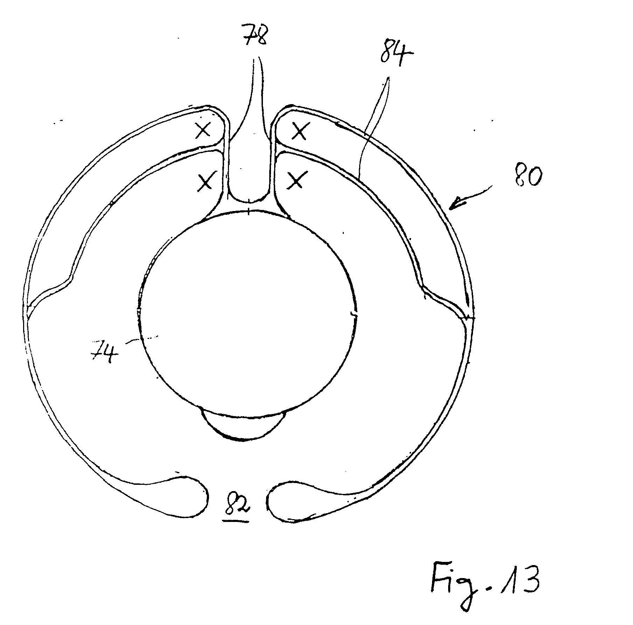

(57) Das Instrument dient dazu, eine Disk-Linse (56) oder Chip-Linse (Fig. 12 und Fig. 13) durch eine zentrale runde Kapsulorhexis in die Linsenkapsel eines Auges zu implantieren. Es weist einen Spannmechanismus auf, der die Linse unter Komprimierung ihres haptischen Teils (60) über im wesentlichen den Linsendurchmesser zu halten geeignet ist. Die Haptikarme (80) einer Chip-Linse werden in dem Spannmechanismus kreuzweise übereinandergefaltet. Die Linse wird in einer Vorschubbewegung aus dem Spannmechanismus gelöst.

Fig. 1

EP 0 436 232 A1

Die Erfindung betrifft ein Instrument zum Implantieren einer künstlichen Linse mit einem zentralen optischen Teil und einem elastisch deformierbaren haptischen Teil in die Linsenkapsel eines Auges.

Bei der operativen Behandlung des Katarakt (grauen Stars) ist es Stand der medizinischen Technik, die Linsenkapsel eines Auges zu öffnen, den getrübten Linsenkern zu entfernen und eine künstliche Linse (Kapselsacklinse) in den Kapselsack zu implantieren. In einer bevorzugten Operationstechnik wird mit einer Kanüle eine zentrale kreisrunde Öffnung (Kapsulorhexis) mit scharfkantigem Rand in die Linsenkapselvorderwand gerissen, der Linsenkern entfernt und eine künstliche Linse durch die Kapsulorhexis in die Linsenkapsel implantiert. Die anatomische Geometrie des Kapselsacks bleibt erhalten, und man hat günstige optische Verhältnisse. Doch lassen sich nur bestimmte Typen von künstlichen Linsen durch eine zentrale runde Kapsulorhexis in die Linsenkapsel einsetzen, da der Linsendurchmesser im Bereich des zur Verankerung dienenden haptischen Teils notwendigerweise größer ist, als der der Kapsulorhexis.

Die meist verwendete künstliche Linse nach Sinskey und Kratz hat einen haptischen Teil in Gestalt zweier in der Linsenhauptebene liegender, gekrümmter Verankerungsbügel, die in zweizählig drehsymmetrischer Anordnung tangential von dem zentralen optischen Teil abstehen und im wesentlichen dessen Randkrümmung folgen. Die Verankerungsbügel lassen sich soweit elastisch deformieren, daß ein Einsetzen der Linse durch eine zentrale runde Kapsulorhexis möglich ist. Nachteilig ist, daß die elastische Spreizung der Verankerungsbügel im Kapselsack nicht homogen über den Umfang des optischen Teils der Linse erfolgt. Es kann zu Verformungen der Linsenkapsel ("American Football") durch die Verankerungsbügel kommen, die eine Fehlausrichtung der künstlichen Linse bedingen und den Operationserfolg in Frage stellen.

Ein bevorzugtes Material für künstliche Linsen ist PMMA (Polymethylmethacrylat), das eine ausgezeichnete physiologische Verträglichkeit, gute optische Eigenschaften und die für den haptischen Teil erwünschte Elastizität hat. Um eine bessere Verformbarkeit der künstlichen Linse für die Implantation zu erreichen, wurde bereits die Verwendung anderer Linsenmaterialien, insbesondere Silikonkautschuk, in Betracht gezogen, doch führt dies nicht zu voll befriedigenden Ergebnissen.

Es gibt bereits künstliche Linsen - sog. Disk-Linsen - aus PMMA, die ein in der Linsenhauptebene liegendes, über den Umfang des optischen Teils geschlossenes haptisches Teil haben. Dieser Aufbau entspricht in idealer Weise der natürlichen Linsengeometrie, doch ist die Implantation von Disk-Linsen in die Linsenkapsel schwierig. Nach dem Stand der Technik wird hierzu die Linsenvorderkapsel mit einem seitlichen, sekantenartig verlaufenden Envelope-Schlitz (Briefkastenschlitz) geöffnet, der Linsenkern entfernt und die Disk-Linse durch den Envelope-Schlitz eingesetzt. Durch den Envelope-Schlitz wird der Kapselsack asymmetrisch abgearbeitet und in seiner anatomischen Geometrie zerstört. Das Kapselsackgewebe ist nicht in der Lage, die Disk-Linse beim Einsetzen durch den Envelope-Schlitz zu deformieren. Es besteht die Gefahr, daß der Envelope-Schlitz beim Einsetzen über den Kapseläquator bis auf die Linsenhinterkapsel durchreißt, worauf eine exakte Positionierung und siohere Verankerung der Disk-Linse in dem Kapselsack nicht mehr gewährleistet ist.

Aus der Praxis sind auch sogenannte Chip-Linsen bekannt. Es handelt sich dabei um Intraokularlinsen mit einem optischen Linsenkörper und einer einstückig damit ausgebildeten Haptik, zu der an einer gemeinsamen Anformstelle mit der Peripherie des Linsenkörpers verbundene, sich in entgegengesetzter Umfangsrichtung mit Abstand um den Linsenkörper herum erstreckende Arme gehören, zwischen deren Enden eine Lücke besteht.

Aufgabe der Erfindung ist es, ein Instrument der eingangs genannten Art zu schaffen, mit dem sich eine vorzugsweise aus PMMA bestehende Disk-Linse oder Chip-Linse bei einhändiger Bedienung durch eine zentrale runde Kapsulorhexis kleineren Durchmessers in die Linsenkapsel eines Auges implantieren läßt.

Diese Aufgabe wird mit einem Instrument gelöst, das einen Spannmechanismus aufweist, der die Linse unter Komprimierung ihres haptischen Teils über im wesentlichen den Linsendurchmesser zu halten geeignet ist und aus dem die Linse in einer Vorschubbewegung lösbar ist.

Durch den Spannmechanismus wird der haptische Teil einer künstlichen Linse derart elastisch deformiert, daß die Linse durch eine zentrale Kapsulorhexis mit wesentlich kleinerem Durchmesser als dem Linsendurchmesser paßt. Die Linse wird in einer Vorschubbewegung aus dem Spannmechanismus gelöst und durch die Kapsulorhexis in die Linsenkapsel implantiert, wobei die Deformation des haptischen Teils dank dessen Elastizität rückgängig gemacht wird und die Linse wieder ihre ursprüngliche Gestalt annimmt.

Die erfindungsgemäße Vorrichtung ermöglicht eine Kataraktoperation, bei der die Linsenkapsel durch eine zentrale runde Kapsulorhexis eröffnet wird, so daß ihre anatomische Geometrie voll erhalten bleibt. Es kann eine in ihren haptischen und optischen Eigenschaften dem natürlichen Linsenkern optimal nachempfundene Linse durch die Kapsulorhexis implantiert werden, ohne daß das Kapselsackgewebe der Gefahr von Beschädigungen unterliegt. Der Durchmesser der Kapsulorhexis braucht im wesentlichen nur dem Durchmesser des optischen Teils der Linse zu entsprechen. Es gelingt beispielsweise, eine Linse mit einem Durchmesser von 9 bis 10 mm durch eine Kapsulorhexis mit einem Durchmesser von 4 bis 6 mm zu implantieren. Der im entspannten Zustand einen deutlich größeren Durchmesser als die Kapsulorhexis aufweisende haptische Teil der Linse gewährleistet eine einwandfreie Positionierung und sichere Verankerung der Linse in dem nicht bzw. nicht nennenswert verformten Kapselsack.

Zu dem Spannmechanismus des erfindungsgemäßen Instruments gehören vorzugsweise zwei einander gegenüberliegende Backen, zwischen die die Linse unter Deformierung ihres haptischen Teils paßt.

In einer ersten Ausführungsform lassen sich die Backen des Spannmechanismus zangenartig spreizen und um die Linse schließen, um diese in dem erforderlichen Maß zu deformieren. Diese in Aufbau und Bedienung allerdings nicht ganz einfache Variante des erfindungsgemäßen Instruments ist universell für verschiedene Linsenformen und -größen verwendbar. Die Linse kann präoperativ auf einfache Weise in den Spannmechanismus eingebracht werden, und man kann das Maß ihrer Verformung von Fall zu Fall individuell bestimmen.

In einer alternativen Ausführungsform des erfindungsgemäßen Instruments sind die Backen starr und wenigstens abschnittsweise aufeinander zu konvergierend gestaltet. Das Instrument weist eine geeignete Haltevorrichtung für die künstliche Linse auf, und es ist so ausgelegt, daß sich die Linse in einer Relativbewegung von Haltevorrichtung und Backen zwischen letztere spannen und daraus lösen läßt. Bei dieser Art Instrument ist man zwar eher auf Linsentyp und -größe festgelegt, und hat auch das Ausmaß der Linsenverformung nicht in so weiten Grenzen in der Hand. Aufbau und Bedienung des Instruments gestalten sich aber sehr einfach.

Die Backen können sich an einem Schieber befinden, der längsverschieblich auf einen Führungsdraht aufgezogen ist. Die Haltevorrichtung für die Linse ist am Ende des Führungsdrahts angeordnet. Als Haltevorrichtung für eine Disk-Linse empfiehlt sich ein am Ende des Führungsdrahts befindlicher Haken, der in eine Positionieröffnung in dem haptischen Teil der Linse paßt.

Eine bevorzugte Haltevorrichtung für eine Chip-Linse besteht aus einer Hakenplatte. Diese kann einen ebenen rechteckigen Plattenkörper und mehrere einseitige davon abstehende Haken aufweisen. Die Hakenplatte übergreift die Anformstelle der Haptik einer Chip-Linse. Ihre Haken kommen hinter dem Ansatz der Haptikarme zum Eingriff, wodurch die Chip-Linse sicher gehalten ist. Eine Halteplatte mit vier im Quadranten angeordneten Haken kommt vorteilhaft für Chip-Linsen zum Einsatz, deren Haptikarme im Bereich der Anformstelle aus zwei radial beabstandeten Bügeln aufgebaut sind. Die Haken hintergreifen den Einsatz der Bügel und halten die Linse drehsicher an vier Punkten.

Das Instrument hat vorzugsweise einen länglichen Griff, bezüglich dessen verlängerter Längsachse der Führungsdraht aufwärts gekrümmt sein kann. Diese Bauform ist handhabungstechnisch bevorzugt.

Der Schieber kann eine im wesentlichen parallel zu dem Führungsdraht sich erstreckende, vorzugsweise ebene Rückenstütze haben, an deren Ende sich eine Rampe befinden kann. Die Backen sind vorzugsweise in symmetrischer Anordnung beidseits der Rückenstütze angeordnet, wobei sie sich bis mindestens auf die Höhe des Führungsdrahts erstrecken. Die Linse ist so allseits zwischen Rückenstütze, Backen und Führungsdraht gehalten.

Die Backen sind vorzugsweise im wesentlichen gerade. Sie können die äußeren Zinken einer dreizinkigen Gabel bilden, deren mittlerer Zinken als Rückenstütze dient.

In einer bevorzugten Ausführungsform haben die Backen ein U-Profil mit einander zugekehrter U-Öffnung. Man gewährleistet so eine sichere Halterung und Führung der Linse zwischen den Backen.

Der U-Rücken der Backen kann mit einem in Backenlängsrichtung sich erstreckenden, an den äußeren Enden der Backen nicht durchgehenden Schlitz versehen sein, durch den der haptische Teil der Linse paßt. Es erfolgt so eine Einschnürung der Linse im wesentlichen zwischen den äußeren Enden der Backen, während sich die Linse über die Länge der Backen zur Seite ausbauchen kann, wobei ihr haptischer Teil durch die Schlitze im Backenrücken nach außen tritt. Die Linse erfährt ihre stärkste Verformung an der Spitze des Instruments, die zur Implantation der Linse an die Kapsulorhexis herangeführt wird. Beim Lösen aus dem Instrument entfalten und entspannen sich die von den Backen freikommenden Linsenpartien in optimaler Weise.

Der Schieber des Instruments kann sich anschlagbegrenzt zwischen einer Einspannposition und Löseposition der Linse verschieben lassen. Durch die Anschlagbegrenzung wird ein wohldefiniertes Maß an Verformung der Linse vorgegeben und die Bedienung des Instruments erleichtert.

Der Schieber kann durch ein Federelement in die Löseposition der Linse vorgespannt sein. Er wird gegen die Kraft des Federelements in die Einspannposition der Linse bewegt, wo sich der Schieber vorzugsweise arretieren läßt. Die Einhandbedienung des Instruments wird damit erleichtert.

Die Erfindung wird im folgenden anhand zweier in den Zeichnungen dargestellter Ausführungsbeispiele näher erläutert. Es zeigen:

Fig. 1 die Draufsicht auf ein Instrument zum Implantieren einer Disk-Linse;

Fig. 2 eine Seitenansicht des Instruments mit Blick in Richtung II von Fig. 1;

Fig. 3 die vergrößerte Draufsicht auf einen Spannmechanismus für die Linse mit Blick in Richtung III von Fig. 2;

Fig. 4 eine Seitenansicht des Spannmechanismus mit Blick in Richtung IV von Fig. 3;

Fig. 5 einen Schnitt durch den Spannmechanismus nach V - V von Fig. 3;

Fig. 6 und Fig. 7 den Spannmechanismus mit einer Disk-Linse in Draufsicht, wobei

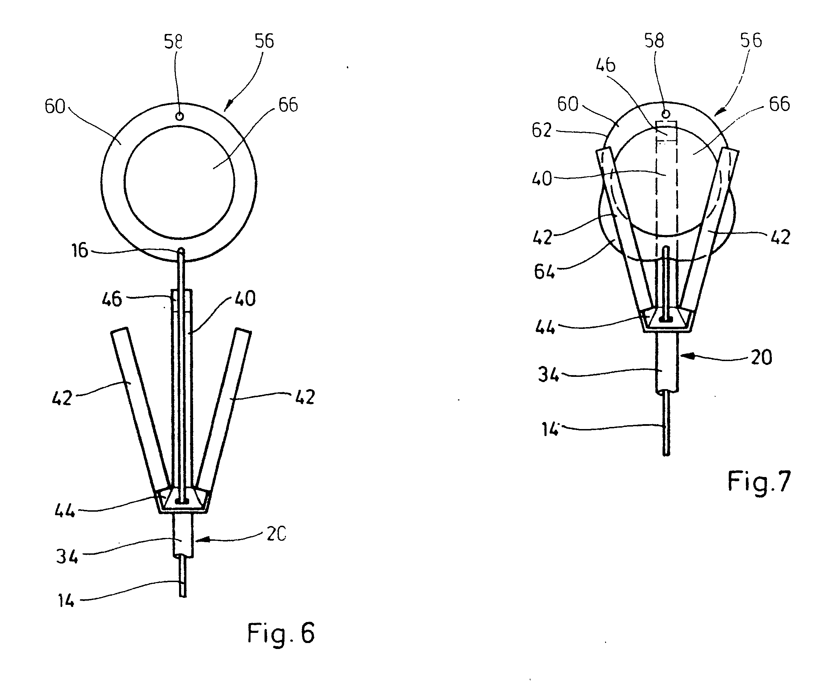

Fig. 6 die Disk-Linse in Lösestellung, und

Fig. 7 in Spannstellung zeigt;

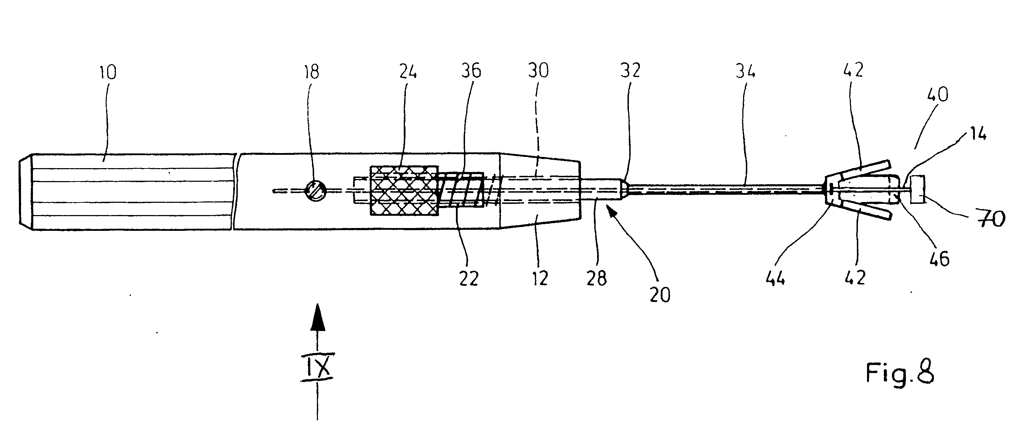

Fig. 8 die Draufsicht auf ein Instrument zum Implantieren einer Chip-Linse

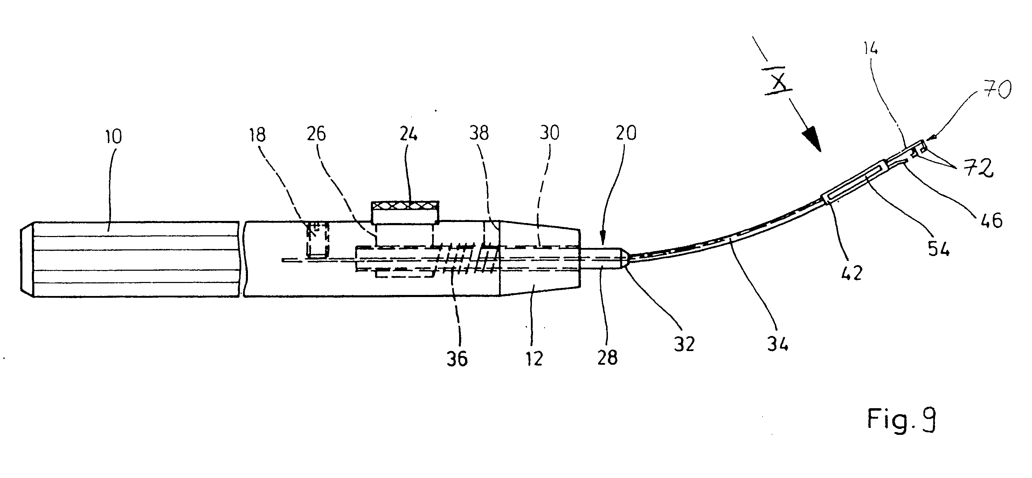

Fig. 9 eine Seitenansicht des Instruments mit Blick in Richtung IX von Fig. 8;

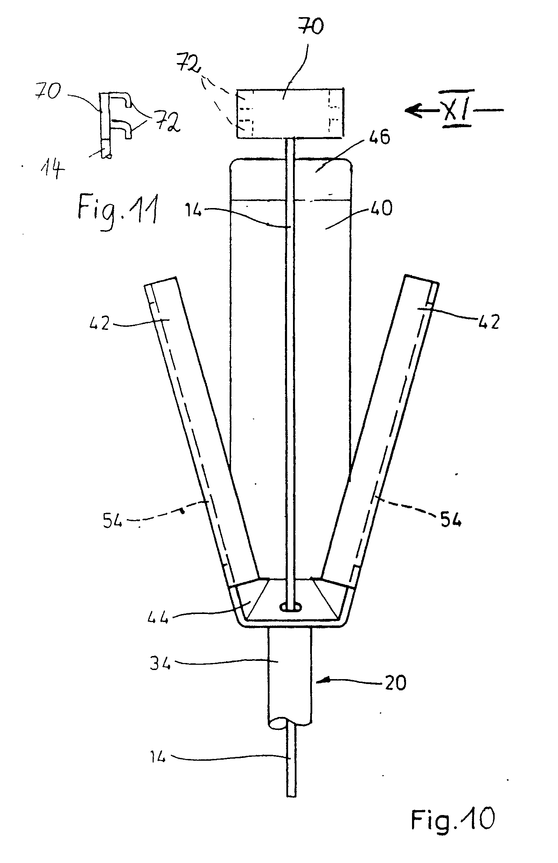

Fig. 10 die vergrößerte Draufsicht auf einen Spannmechanismus für die Linse mit Blick in Richtung X von Fig. 9;

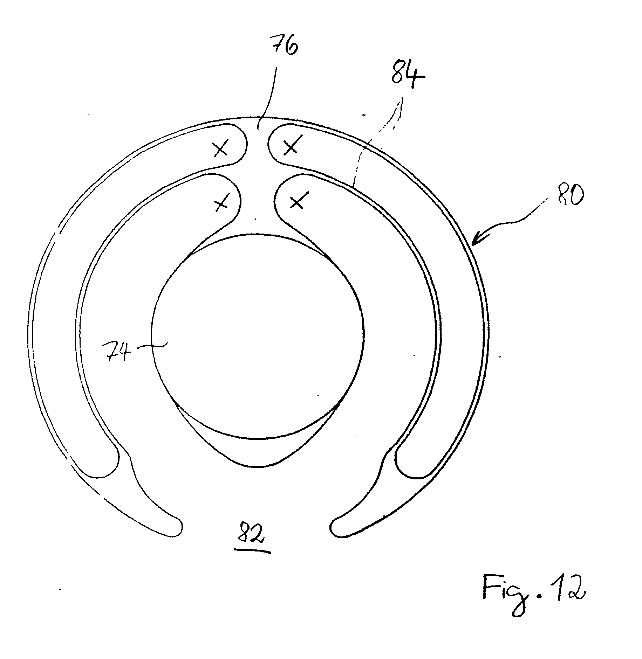

Fig. 11 die Seitenansicht einer zu dem Spannmechanismus gehörenden Haltevorrichtung mit Blick in Richtung XI von Fig. 10; und

Fig. 12 und Fig. 13 mit dem Instrument zu implantierende Chip-Linsen in Draufsicht.

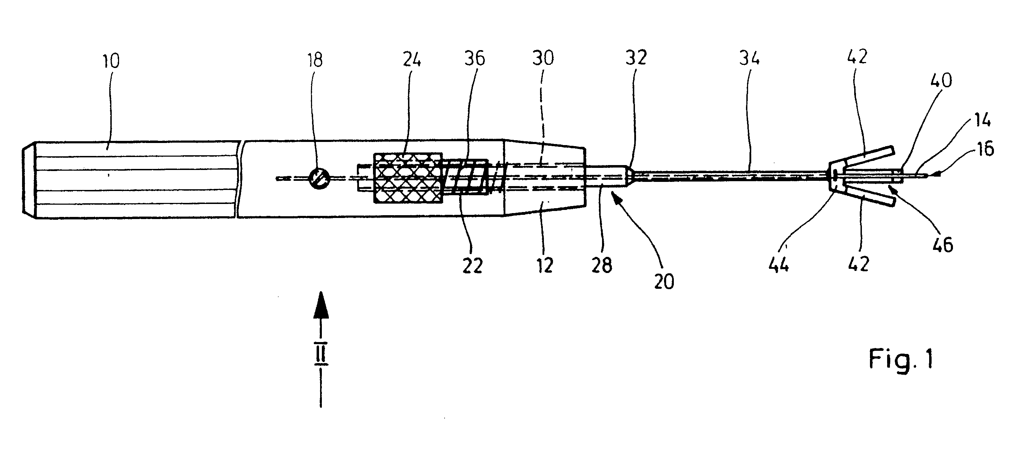

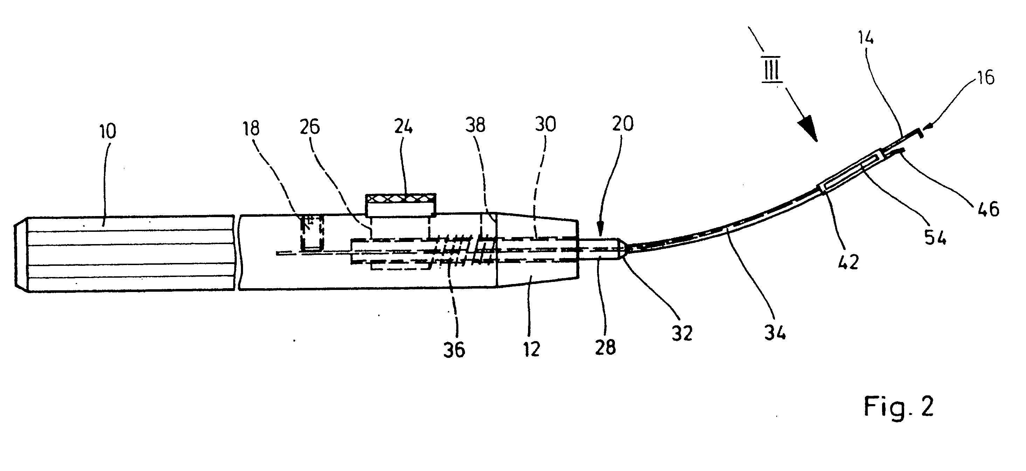

Bezugnehmend auf Fig. 1 und 2, hat das dargestellte Instrument einen stabförmigen Handgriff 10, dessen Querschnitt rund oder polygonal gestaltet sein kann und der sich zu seinem vorderen Ende 12 konisch verjüngt. Der Griff 10 ist hohl. Er enthält einen Führungsdraht 14, der sich im wesentlichen auf der Längsmittelachse des Griffs 10 über dessen vordere Hälfte erstreckt und aus einer Öffnung an der vorderen Stirnseite des Griffendes 12 austritt. Der Führungsdraht 14 folgt dann einer Verlängerung der Griffachse und ist im weiteren Verlauf aufwärts gekrümmt. Das Ende des Führungsdrahts 14 bildet einen Haken 16. Der Führungsdraht 14 ist so steif, daß er seine Form auf Dauer beibehält. Er ist mit einer Spannschraube 18 starr an dem Griff 10 befestigt.

Auf den Führungsdraht 14 ist in Teleskopanordnung längsverschieblich ein Schieber 20 aufgezogen, zu dem ein in dem Griff 10 aufgenommenes Schieberteil 26 gehört. Der Griff 10 ist mit einem in Grifflängsrichtung verlaufenden Langloch 22 versehen. Er trägt an seiner Außenseite einen Betätigungsknopf 24, der durch das Langloch hindurch mit dem im Innern des Griffs 10 befindlichen Schieberteil 26 fest verbunden ist. An dem Schieberteil 26 sitzt eine Führungshülse 28, die den Führungsdraht 14 umschließt und in einer axialen Längsbohrung 30 des Griffs 10 geführt ist. Letztere bildet die erwähnte Öffnung an der vorderen Stirnseite des Griffendes 12, aus der der Führungsdraht 14 und die darauf aufgezogene Führungshülse 28 austreten, wobei letztere etwas Überstand hat. Die Führungshülse 28 geht an einer Schulter 32 in ein fest daran sitzendes, biegsames Rohr 34 kleineren Durchmessers über, das auf den Führungsdraht 14 aufgezogen ist und dessen Krümmung folgt. Am Ende des Rohres 34 befindet sich ein damit fest verbundener Spannmechanismus.

Der Griff 10 enthält eine um die Führungshülse 28 herumliegende Schraubendruckfeder 36, die sich einends am vorderen Ende des Schieberteils 26, und andernends an einer im Innern des Griffs 10 vor der Führungsbohrung 30 befindlichen Schulter 38 abstützt. Die Feder 36 spannt den Schieber 20 in die in Fig. 1 und 2 gezeigte zurückgezogene Stellung. Der Schieber kann mittels des Betätigungsknopfes 24 gegen die Kraft der Feder 36 auf dem Führungsdraht 14 vorgeschoben werden, wodurch der Spannmechanismus und der am Ende des Führungsdrahts 14 befindliche Haken 16 eine Verschiebebewegung relativ zueinander ausführen. Das bestehende Verschiebespiel ist durch die Länge des Langlochs 22 vorgegeben, an dessen Enden der Betätigungsknopf 24 Anschlagstellungen einnimmt. In der Anschlagstellung, in der der Schieber 20 maximal vorgeschoben und die Druckfeder 36 gespannt ist, besteht eine Arretiermöglichkeit mit einer nicht näher dargestellten, manuell betätigten Arretiereinrichtung.

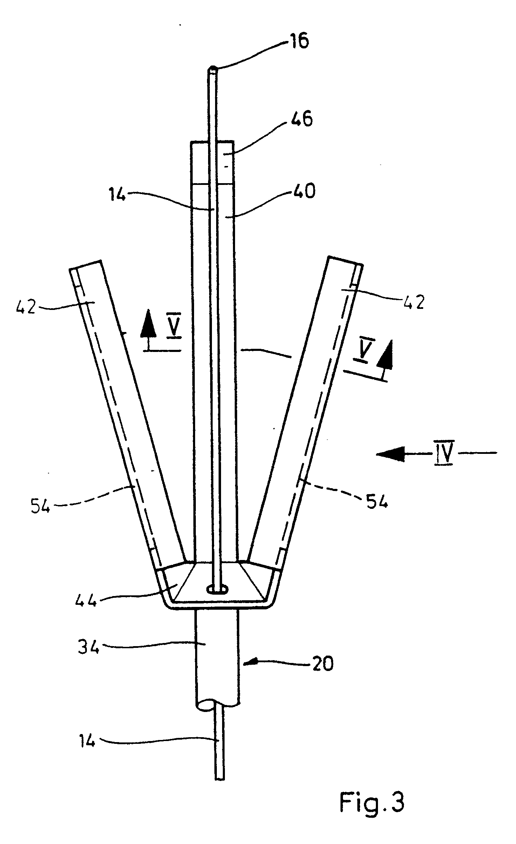

Der Aufbau des Spannmechanismus kann im einzelnen Fig. 3 bis 5 entnommen werden. Am Ende des Rohrs 34 sitzt fest eine dreizinkige Gabel mit geraden Zinken, deren mittlerer Zinken 40 im wesentlichen eine gerade Verlängerung des Rohrs 34 bildet, während die äußeren Zinken 42 in symmetrischer Anordnung beidseits des mittleren Zinkens 40 zu liegen kommen und sich von dem Scheitel 44 der Gabel, an dem sich die Zinken 42, 44 treffen, geradlinig schräg nach außen erstrekken. Der zwischen dem mittleren und den äußeren Zinken 40, 42 eingeschlossene Winkel beträgt ca. 15°.

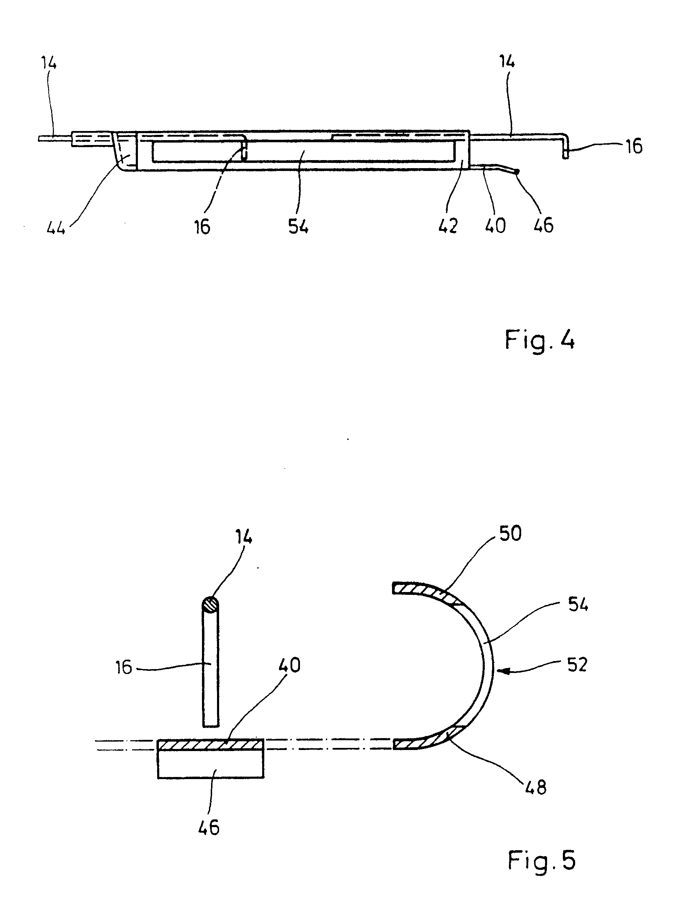

Der mittlere Zinken 40 ist im wesentlichen eben und an seinem Ende, das über die äußeren Zinken 42 vorsteht, mit einer nach unten abgewinkelten Rampe 46 versehen. Der Führungsdraht 14 tritt oberhalb des mittleren Zinkens 40 aus dem Scheitel 44 der Gabel aus und erstreckt sich parallel und im Abstand über den mittleren Zinken 40. Der Haken 16 am Ende des Führungsdrahts 14 ist unter 90° nach unten abgewinkelt. In der zurückgezogenen Anschlagstellung des Schiebers 20 kommt der Haken 16 in einigem Abstand vor der Rampe 46 des mittleren Zinkens 40 zu liegen (Darstellung mit durchgezogener Linie in Fig. 4). In der vorgeschobenen Anschlagstellung des Schiebers 20 befindet sich der Haken 16 zwischen den äußeren Zinken 42 dicht vor dem Scheitel 44 der Gabel (gestrichelte Darstellung in Fig. 4).

Die äußeren Zinken 42 der Gabel sind als Backen gestaltet, zwischen die eine künstliche Linse unter elastischer Deformierung ihres haptischen Teils eingespannt werden kann. Die Backen haben ein U-Profil mit einander zugewandten U-Öffnungen. Der untere U-Schenkel 48 des U-Profils liegt in einer Ebene mit dem mittleren Zinken 40, während sich der obere U-Schenkel 50 etwas oberhalb des Führungsdrahts 14 erstreckt. Der U-Rücken 52 des Profils ist mit einem in Backenlängsrichtung verlaufenden Schlitz 54 versehen, der vom Scheitelbereich 44 der Gabel ausgeht und bis dicht vor die Enden der Backen 42 reicht. Die Schlitze 54 sind so dimensioniert, daß der haptische Teil einer künstlichen Linse hindurchpaßt. Ein Maximum an Verformung erfährt der haptische Teil an den ungeschlitzten Enden der Backen, deren Abstand etwa dem Durchmesser des optischen Teils der künstlichen Linse entspricht.

In Fig. 6 und Fig. 7 ist die Handhabung einer Disk-Linse 56 mit dem erfindungsgemäßen Instrument illustriert. In der zurückgeschobenen Ausgangsstellung des Schiebers 20 wird der Haken 16 in eine Positionieröffnung 58 im haptischen Teil 60 der Disk-Linse 56 eingehakt. Der Schieber 20 wird vorgeschoben und die Linse 56 mit dem mittleren Zinken 40 der Gabel aufgenommen. Das wird durch die Rampe 46 erleichtert. Die Linse 56 wird zwischen den Backen 42 und dem als Rückenstütze dienenden mittleren Zinken 40 der Gabel eingespannt und an den Backenenden elastisch so deformiert, daß sich eine Einschnürung des haptischen Teils 60 im wesentlichen über den Durchmesser der Linse 56 ergibt. Vor den Backenenden bilden sich kleinere, und hinter den Backenenden größere Bäuche 62, 64 des haptischen Teils 60, wobei letztere 64 durch die Schlitze 54 der Backen 42 nach außen vorstehen. Sobald die vorgeschobene Anschlagstellung des Schiebers 20 gemäß Fig. 7 erreicht ist, erfolgt eine Arretierung.

Die Linse 56 kann nun mit dem Instrument durch eine zentrale runde Kapsulorhexis in die Linsenkapsel eines Auges implantiert werden, wobei der Durchmesser der Kapsulorhexis im wesentlichen dem des optischen Teils 66 der Linse 56 entspricht. Die Linse 56 wird mit den vorderen kleineren Bäuchen 62 des haptischen Teil 60 voran durch durch die Kapsulorhexis eingeführt und nach Lösen der Arretierung durch Zurückziehen des Schiebers 20 aus ihrer Spannstellung freigegeben, wobei sie in ihre ursprüngliche Form zurückkehrt. Eine exakte Positionierung der Linse 56 in der Linsenkapsel erfolgt mit dem Haken 16, der abschließend von dem haptischen Teil 60 gelöst wird.

Fig. 8 bis 11 zeigen ein Instrument zum Implantieren einer Chip-Linse. Gleiche Teile sind mit übereinstimmenden Bezugszeichen versehen, und die vorstehende Beschreibung gilt sinngemäß entsprechend. Das Instrument hat als Haltevorrichtung für die Chip-Linse eine am Ende des Führungsdrahts sitzende Hakenplatte 70 mit einem ebenen rechteckigen Plattenkörper, dessen Breite etwa der des mittleren Zinkens 42 entspricht. An den Ecken des Plattenkörpers 70 befinden sich vier nach unten abgewinkelte, rückwärts gekrümmte Haken 72. In der zurückgezogenen Anschlagstellung des Schiebers 20 kommt die Hakenplatte 70 in einigem Abstand vor der Rampe 46 des mittleren Zinkens 40 zu liegen. In der vorgeschobenen Anschlagstellung des Schiebers 20 befindet sich die Hakenplatte 70 zwischen den äußeren Zinken 42 dicht vor dem Scheitel 44 der Gabel.

Bei einem Instrument mit Hakenplatte 70 ist verglichen mit dem zuvor beschriebenen Instrument mit Haken eine breitere Ausbildung des mittleren Zinkens 40 bevorzugt, da eine zu implantierende Chip-Linse mit offener Rundhaptik beim Einspannen einer flächigeren Abstützung bedarf.

In Fig. 12 und Fig. 13 sind Chip-Linsen dargestellt, die mit dem erfindungsgemäßen Instrument implantiert werden. Die Linsen haben einen zentralen optischen Linsenköper 74 und eine einstückig damit ausgebildete Haptik. In der Bauvariante gemäß Fig. 12 geht an der Anformstelle der Haptik von der Peripherie des Linsenkörpers 74 ein massiver radialer Steg 76 ab, während in der Bauvariante gemäß Fig. 13 zwei parallele dünne Stege 78 mit annähernd radialer Erstreckung vorgesehen sind. Von den Stegen 76, 78 gehen in entgegengesetzter Umfangsrichtung mit Abstand um den Linsenkörper 74 herum sich erstreckende Haptikarme 80 ab, die nicht ganz über den Umfang geschlossen sind, sondern zwischen ihren Enden eine Lücke 82 lassen. Die Arme 80 sind zumindest im Bereich der Anformstelle aus je zwei parallel von den Stegen 76, 78 abgehenden, radial beabstandeten Bügeln 84 aufgebaut. Die Hakenplatte 70 ist so dimensioniert, daß sie die Stege 76, 78 übergreift und mit den Haken 72 am Ansatz der Bügel 84 zum Eingriff kommt. Die Eingriffspunkte sind in Fig. 12 und Fig. 13 durch ein Kreuz gekennzeichnet.

Zur Implantation einer Chip-Linse mit dem erfindungsgemäßen Instrument wird in der zurückgeschobenen Ausgangsstellung des Schiebers 20 die Hakenplatte 70 an der Haptik der Linse eingehakt. Der Schieber 20 wird vorgeschoben und die Linse mit dem mittleren Zinken 40 der Gabel aufgenommen. Die Linse wird zwischen den Backen 42 und dem als Rückenstütze dienenden mittleren Zinken 40 der Gabel eingespannt und an den Backenenden elastisch so deformiert, daß sich die Haptikarme symmetrisch übereinanderfalten und kreuzen. Dadurch wird die Linse sowohl in der Breite, als auch in der Länge zur Implantation optimal verkleinert. Sobald die vorgeschobene Anschlagstellung des Schiebers 20 erreicht ist, erfolgt eine Arretierung.

Die Linse 56 kann nun mit dem Instrument durch eine zentrale runde Kapsulorhexis in die Linsenkapsel eines Auges implantiert werden, wobei der Durchmesser der Kapsulorhexis im wesentlichen dem des optischen Linsenkörpers 74 entspricht. Die Linse wird mit den übereinandergefalteten Haptikarmen 80 voran durch die Rhexisöffnung in den Kapselsack eingeführt und nach Lösen der Arretierung durch Zurückziehen des Schiebers 20 aus ihrer Spannstellung freigegeben, wobei sie in ihre ursprüngliche Form zurückkehrt. Eine exakte Positionierung der Linse in der Linsenkapsel erfolgt mit dem Hakenplatte 70, die abschließend von der Haptik gelöst wird. Liste der Bezugszeichen

| 10 | Handgriff |
| 12 | Griffende |
| 14 | Führungsdraht |
| 14' | Führungsdraht |
| 16 | Haken |
| 16' | Haken |
| 18 | Spannschraube |
| 20 | Schieber |
| 22 | Langloch |
| 24 | Betätigungsknopf |
| 26 | Schieberteil |
| 28 | Führungshülse |
| 30 | Bohrung |
| 32 | Schulter |
| 34 | Rohr |
| 36 | Schraubendruckfeder |
| 38 | Schulter |
| 40 | mittlerer Zinken |
| 42 | äußerer Zinken |
| 44 | Scheitel |
| 46 | Rampe |
| 48 | unterer U-Schenkel |
| 50 | oberer U-Schenkel |
| 52 | U-Rücken |
| 54 | Schlitz |
| 56 | Disk-Linse |
| 58 | Positionieröffnung |
| 60 | haptischer Teil |
| 62 | kleiner vorderer Bauch |
| 64 | großer hinterer Bauch |
| 66 | optischer Teil |
| 70 | Hakenplatte |
| 72 | Haken |
| 74 | Linsenkörper |
| 76 | Steg |
| 78 | Doppelsteg |
| 80 | Arm |
| 82 | Lücke |
| 84 | Bügel |

## Ansprüche

1. Instrument zum Implantieren einer künstlichen Linse mit einem zentralen optischen Teil und einem elastisch deformierbaren peripheren haptischen Teil in die Linsenkapsel eines Auges, dadurch gekennzeichnet, daß es einen Spannmechanismus aufweist, der die Linse (56) unter Komprimierung ihres haptischen Teils (60) über im wesentlichen den Linsendurchmesser zu halten geeignet ist und aus dem die Linse (56) in einer Vorschubbewegung lösbar ist.

2. Instrument nach Anspruch 1, dadurch gekennzeichnet, daß zu dem Spannmechanismus zwei einander gegenüberliegende Backen (52) gehören, zwischen die die Linse (56) unter Deformierung ihres haptischen Teils (60) paßt.

3. Instrument nach Anspruch 1 oder 2, dadurch gekennzeichnet, daß die Backen zangenartig aufspreizbar und um die Linse (56) schließbar sind.

4. Instrument nach einem der Ansprüche 1 bis 3, dadurch gekennzeichnet, daß die Backen (42) starr und wenigstens abschnittsweise aufeinander zu konvergierend gestaltet sind, und daß eine Haltevorrichtung für die Linse (56) vorgesehen und die Linse (56) in einer Relativbewegung von Haltevorrichtung und Backen (42) zwischen letztere spannbar und daraus lösbar ist.

5. Instrument nach einem der Ansprüche 1 bis 4, dadurch gekennzeichnet, daß sich die Backen (42) an einem Schieber (20) befinden, der längsverschieblich auf einen Führungsdraht (14) aufgezogen ist, und daß die Haltevorrichtung am Ende des Führungsdrahts (14) ausgeordnet ist.

6. Instrument nach einem der Ansprüche 1 bis 5, dadurch gekennzeichnet, daß die Haltevorrichtung aus einem Haken (16) oder einer Hakenplatte (70) besteht.

7. Instrument nach Anspruch 6, dadurch gekennzeichnet, daß die Hakenplatte (70) einen ebenen, vorzugsweise rechteckigen Plattenkörper und mehrere einseitig davon abstehende Haken (72) aufweist, insbesondere vier im Quadranten angeordnete Haken (72).

8. Instrument nach einem der Ansprüche 1 bis 7, dadurch gekennzeichnet, daß es einen länglichen Griff (10) hat, bezüglich dessen verlängerter Längsachse der Führungsdraht (14) vorzugsweise aufwärts gekrümmt ist.

9. Instrument nach einem der Ansprüche 1 bis 8, dadurch gekennzeichnet, daß der Schieber (20) eine im wesentlichen parallel zu dem Führungsdraht (14) sich erstreckende, vorzugsweise ebene Rückenstütze (40) hat, an deren Ende sich eine Rampe (46) befinden kann, und daß die Backen (42) in symmetrischer Anordnung beidseits der Rückenstütze (40) angeordnet sind und sich bis mindestens auf die Höhe des Führungsdrahts (14) erstrecken.

10. Instrument nach einem der Ansprüche 1 bis 9, dadurch gekennzeichnet, daß die Backen (42) im wesentlichen gerade sind und die äußeren Zinken einer dreizinkigen Gabel bilden, deren mittlerer Zinken als Rückenstütze (40) ausgebildet ist.

11. Instrument nach einem der Ansprüche 1 bis 10, dadurch gekennzeichnet, daß die Backen (42) ein U-Profil mit einander zugekehrter U-Öffnung haben.

12. Instrument nach einem der Ansprüche 1 bis 11, dadurch gekennzeichnet, daß der U-Rükken (52) der Backen (42) mit einem in Backenlängsrichtung sich erstreckenden, an den äußeren Enden der Backen (42) nicht durchgehenden Schlitz (54) versehen ist, durch den der haptische Teil (60) der Linse (56) paßt.

13. Instrument nach einem der Ansprüche 1 bis 12, dadurch gekennzeichnet, daß der Schieber (20) anschlagbegrenzt zwischen einer Einspannposition und Löseposition der Linse (56) verschieblich ist.

14. Instrument nach einem der Ansprüche 1 bis 13, dadurch gekennzeichnet, daß der Schieber (20) durch ein Federelement (36) in die Löseposition der Linse (56) vorgespannt ist.

15. Instrument nach einem der Ansprüche 1 bis 14, dadurch gekennzeichnet, daß der Schieber (20) in der Einspannposition der Linse (56) arretierbar ist.

Fig. 1

Fig. 2

EP 0 436 232 A1

Fig.3

Fig. 4

Fig. 5

Fig.7

Fig.6

Fig.8

EP 0 436 232 A1

Fig. 9

EP 0 436 232 A1

Fig.11

Fig.10

Fig. 12

Fig. 13

| | EINSCHLÄGIGE DOKUMENTE | | EP 90125806.1 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | <u>FR - A1 - 2 604 896</u><br>(MAZZOCCO)<br>  * Zusammenfassung; Seite 1,<br>    Zeile 1 - Seite 4, Zeile 19;<br>    Anspruch 1; Fig. 35,57 *<br>    -- | 1 | A 61 F 9/00 |
| A | <u>FR - A1 - 2 631 545</u><br>(BIODOMI)<br>  * Zusammenfassung; Seite 1,<br>    Zeile 1 - Seite 2, Zeile 16;<br>    Anspruch 1; Fig. 3 *<br>    ---- | 1 | |
| | | | **RECHERCHIERTE SACHGEBIETE (Int Cl⁵)**<br><br>A 61 F<br>A 61 B |

Der vorliegende Recherchenbericht wurde für alle Patentansprüche erstellt.

| Recherchenort | Abschlußdatum der Recherche | Prüfer |
|---|---|---|
| WIEN | 25-03-1991 | GRÖSSING |